# EUROPEAN PATENT APPLICATION

(11) **EP 4 714 543 A1**
(43) Date of publication of application: **25.03.2026**
(21) Application number: 24813104.7
(22) Date of filing: 31.05.2024
(51) Int. Cl.: B01J 35/54, B01J 19/24, B01J 23/63, B01J 23/83, B01J 23/755, B01J 35/60, C01B 3/40, C01B 5/00, C01B 32/40, C07B 61/00, C07C 1/12, C07C 9/04

(54) **STRUCTURE CATALYST, METHOD FOR PRODUCING COMPOUND, AND REACTION DEVICE**

(30) Priority: 02.06.2023 JP 2023091756
(71) Applicant: NATIONAL UNIVERSITY CORPORATION SHIZUOKA UNIVERSITY, Shizuoka-shi, Shizuoka 422-8529 (JP)
(72) Inventor: FUKUHARA, Choji, Hamamatsu-shi, Shizuoka 432-8561 (JP); AKAMA, Hiroshi, Hamamatsu-shi, Shizuoka 432-8561 (JP); WATANABE, Ryo, Hamamatsu-shi, Shizuoka 432-8561 (JP)
(74) Representative: Hoffmann Eitle
(86) International application number: PCT/JP2024/020102
(87) International publication number: WO 2024/248149

(57) **Abstract**

There is disclosed a structure catalyst including a base component that extends along a fixed axis and a catalyst layer that is provided on the base component. The base component has a porous metal portion including the outer surface of the base component, and the catalyst layer is adhered to the porous metal portion. The base component has a plate-like portion that extends along the axis while being twisted in a direction of rotation about the axis.

## Description

### Technical Field

The present disclosure relates to a structure catalyst, a method for producing a compound, and a reaction device.

### Background Art

Conventionally, the production of various compounds according to a gas-solid phase reaction in the presence of a catalyst provided on a base component has been examined (for example, Patent Literature 1).

### Citation List

### Patent Literature

[Patent Literature 1] Japanese Unexamined Patent Publication No. 2020-033280

### Summary of Invention

### Technical Problem

The present disclosure relates to obtaining a desired compound with high efficiency according to a gas-solid phase reaction in the presence of a catalyst.

### Solution to Problem

The present disclosure includes the following aspects.
[1] A structure catalyst, including
   a base component that extends along a fixed axis; and
   a catalyst layer that is provided on the base component,
   wherein the base component has a porous metal portion including the outer surface of the base component, and the catalyst layer is adhered to the porous metal portion, and
   the base component has a plate-like portion that extends along the axis while being twisted in a direction of rotation about the axis.
[2] The structure catalyst according to [1], wherein the mass of the catalyst layer per 1 L of the volume of the base component is 1.0 g or more.
[3] The structure catalyst according to [1] or [2], wherein the average pore diameter of the porous metal portion is 0.1 mm or more.
[4] The structure catalyst according to any one of [1] to [3], wherein the catalyst layer covers the entire outer surface of the base component.
[5] A method for producing a compound, including producing a desired compound from a raw material gas through to a gas-solid phase reaction in a reaction tube in which the structure catalyst according to any one of [1] to [4] is provided,
   wherein the structure catalyst is inserted into the reaction tube in an orientation such that the axis is parallel to a longitudinal direction of the reaction tube.
[6] A reaction device, including:
   a reaction tube; and
   the structure catalyst according to any one of [1] to [4] accommodated in the reaction tube,
   wherein the structure catalyst is inserted into the reaction tube in an orientation such that the axis is parallel to a longitudinal direction of the reaction tube.

### Advantageous Effects of Invention

It is possible to obtain a desired compound with high efficiency according to a gas-solid phase reaction in the presence of a catalyst. It is also possible to perform a continuous gas-solid phase reaction with low pressure loss.

### Brief Description of Drawings

FIG. 1 is a schematic view showing an example of a reaction device.
FIG. 2 is a schematic view showing an example of a structure catalyst.
FIG. 3 is an end view taken along the line III-III in FIG. 2.
FIG. 4 is an image of an example of a produced structure catalyst.
FIG. 5 is a graph showing the relationship between a CO₂ conversion and a CO₂ concentration in a raw material gas in a methanation reaction test.
FIG. 6 is a graph showing the relationship between a CO₂ conversion and a CO₂ concentration in a raw material gas in a methanation reaction test.
FIG. 7 is a graph showing the relationship between a CO₂ conversion and a CO₂ concentration in a raw material gas in a methanation reaction test.
FIG. 8 is a graph showing the relationship between a CO₂ conversion and a set temperature of an electric furnace in a methanation reaction test.
FIG. 9 is a graph showing the relationship between a pressure loss and a set temperature of an electric furnace in a methanation reaction test.
FIG. 10 is a graph showing the relationship between a CH₄ conversion and the temperature in a dry reforming reaction test.
FIG. 11 is a graph showing the relationship between a CO₂ conversion and the temperature in a dry reforming reaction test.
FIG. 12 is a graph showing the relationship between a CO₂ conversion and the temperature in a reverse shift reaction test.

### Description of Embodiments

The present invention is not limited to the following examples.

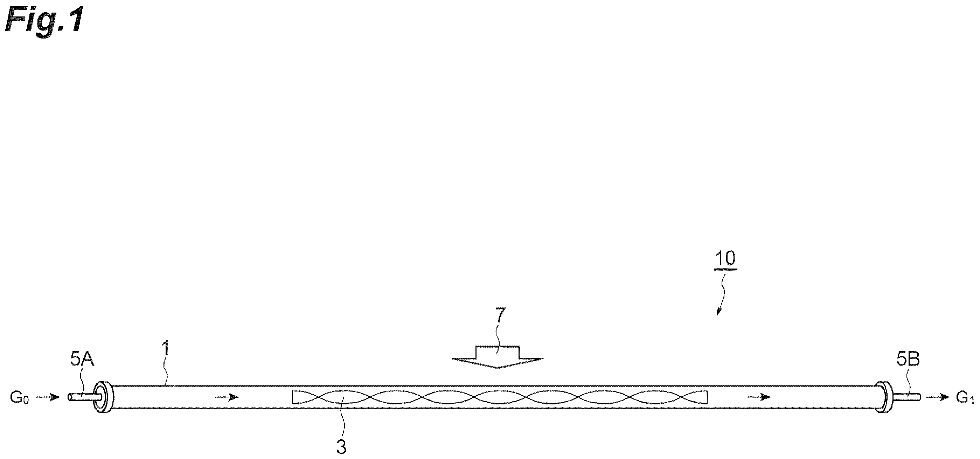

An example of a method for producing a compound according to the present disclosure includes producing a desired compound from a raw material gas according to a gas-solid phase reaction in a reaction tube in which a structure catalyst is provided. FIG. 1 is a schematic view showing an example of a reaction device used to produce a compound. A reaction device 10 shown in FIG. 1 includes a reaction tube 1, a structure catalyst 3 accommodated in the reaction tube 1, and a gas inlet 5A and a gas outlet 5B provided at both ends of the reaction tube 1, respectively. A raw material gas G₀ containing a starting material is introduced into the reaction tube 1 from the gas inlet 5A. In the raw material gas in the reaction tube 1, a gas-solid phase reaction proceeds due to the catalytic action of the structure catalyst 3. A product gas G₁ containing a desired compound, which is the product of the gas-solid phase reaction, is discharged from the gas outlet 5B.

The reaction tube 1 is heated by supplying heat from an externally provided heat source 7. The heat source 7 is usually provided around the reaction tube 1. The heat source 7 is not particularly limited, and for example, it may be an electric heater that generates heat by resistance heating or the like or a heat medium heated to a predetermined temperature. The temperature of the heat source 7 can be adjusted such that, for example, it is within a range of 5°C or higher and 1,000°C or lower in consideration of the type of the gas-solid phase reaction and the like.

The length of the reaction tube 1 may be, for example, 10 to 20,000 mm. The inner diameter of the reaction tube 1 may be, for example, 5 to 200 mm. A plurality of reaction tubes 1 may be provided. The inner surface of the reaction tube 1 may be non-porous. The porosity of the portion forming the non-porous inner surface of the reaction tube 1 may be 10% or less.

The gas-solid phase reaction may include, for example, a methanation reaction through which methane is generated from carbon dioxide and hydrogen according to the following reaction. In the case of a methanation reaction, the raw material gas contains carbon dioxide and hydrogen, and the product gas contains methane as a desired compound and water. In consideration of efficient reaction and the like, the raw material gas may further contain oxygen.

CO₂+4H₂→CH₄+2H₂O

The gas-solid phase reaction applied to the method according to the present disclosure is not limited to the methanation reaction. Other examples of gas-solid phase reactions include a dehydrogenation reaction, a hydrogenation reaction, a steam reforming reaction, a dry reforming reaction, a water-gas shift reaction, a reverse shift reaction, an ammonia synthesis reaction, an ammonia decomposition reaction and a denitrification reaction.

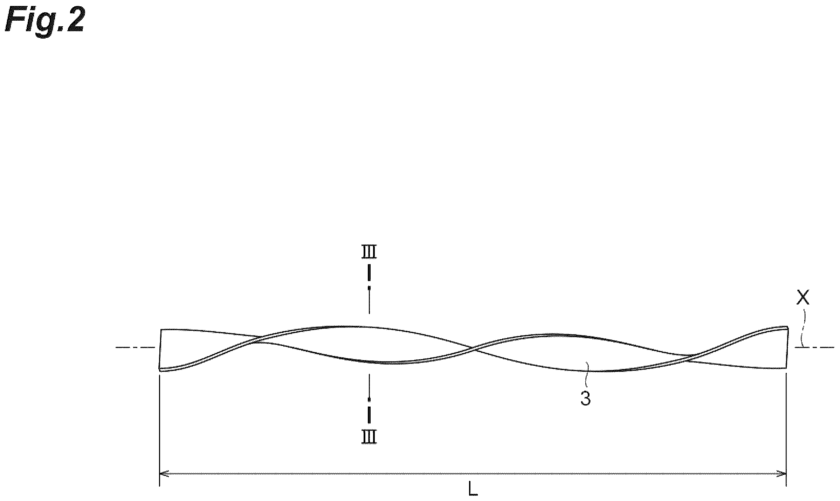

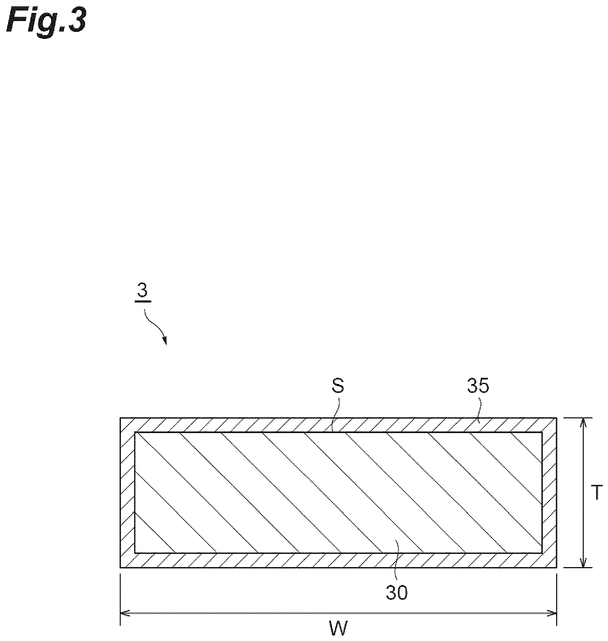

FIG. 2 is a schematic view showing an example of a structure catalyst, and FIG. 3 is an end view taken along the line **III-III** in FIG. 2. The structure catalyst 3 shown in FIG. 2 and FIG. 3 includes a base component 30 having a portion that extends along a fixed axis X and a catalyst layer 35 covering the outer surface S of the base component 30. In the base component 30, the axis X is also the center line of the base component 30. In the example of FIG. 3, the catalyst layer 35 covers the entire outer surface of the base component 30, but the catalyst layer 35 does not need to cover the entire outer surface of the base component 30. The base component 30 as a whole is composed of a plate-like portion that extends along the axis X while being twisted in a direction of rotation about the axis X. In this specification, this type of shape is sometimes referred to as a "spiral type." In the reaction device 10 in FIG. 1, the structure catalyst 3 is inserted into the reaction tube 1 in an orientation such that the axis X is parallel to the longitudinal direction of the reaction tube 1. Two or more structure catalysts 3 may be inserted in series into one reaction tube. Since the structure catalyst 3 inserted into the reaction tube 1 is not integrated with the reaction tube, it can be easily removed from the reaction tube 1. In this regard, the structure catalyst 3 is advantageous compared to a catalyst provided on a plate-like member provided to protrude convexly from the inner surface of the reaction tube. The structure catalyst 3 may be provided apart from the inner surface of the reaction tube 1.

The base component 30 or its plate-like portion has a porous metal portion including the outer surface of the base component 30. The entire base component 30 may be a porous metal portion, or the base component 30 may have a porous metal portion positioned at the outermost layer and a non-porous internal structure provided inside the porous metal portion. The catalyst layer 35 is adhered to the porous metal portion. A part of the catalyst layer 35 may penetrate into the pores of the porous metal portion. In other words, the catalyst layer 35 may have a portion that penetrates into the pores of the porous metal portion and a portion that is provided outside the porous metal portion.

When the amount of the catalyst layer 35 adhered to the base component 30 in the structure catalyst 3 is large, the gas-solid phase reaction tends to proceed particularly efficiently. For example, the mass of the catalyst layer 35 adhered to the base component 30 may be 1 g or more and 1,000 g or less per 1 L of the volume of the base component. The mass of the catalyst layer 35 adhered to the base component 30 may be 10 g or more, 20 g or more, 30 g or more, 40 g or more, or 50 g or more per 1 L of the volume of the base component. The base component 30 having a porous metal portion makes it possible to easily increase the amount of the catalyst layer 35 adhered. The volume of the base component here also includes the volume of the pores in the porous metal portion. Here, in this specification, the volume of the base component is the volume of the cylindrical space formed by the base component processed into a spiral shape. For example, the volume of the spiral-type base component with a width of 100 mm and a length of 100 mm is 0. 785 L.

The porous metal portion may contain pores that communicate with each other. The average pore diameter of the porous metal portion may be, for example, 1 µm or more and 100 mm or less. The average pore diameter here is the average value of the pore diameters of the plurality of pores constituting the porous metal portion. The maximum width of the pores can be regarded as the pore diameter of the pores. The average pore diameter can be, for example, the average value of the pore diameters of 10 or more arbitrary pores. The average pore diameter of the porous metal portion may be 5 µm or more, 10 µm or more, 50 µm or more, 0.1 mm or more, 0. 2 mm or more, 0.3 mm or more, or 0. 4 mm or more, and may be 50 mm or less, 40 mm or less, 30 mm or less, 20 mm or less, 10 mm or less, 5 mm or less, 4 mm or less, 3 mm or less, 2 mm or less, or 1 mm or less.

The porosity in the porous metal portion may be, for example, 40% or more and 99% or less. The porosity is the proportion of the volume of the pores based on the total volume of the porous metal portion including the volume of the pores. The porosity in the porous metal portion may be 45% or more, 50% or more, 55% or more, 60% or more, 65% or more, 70% or more, 75% or more, 80% or more, or 90% or more, and may be 95% or less, 90% or less, 85% or less, 80% or less, 75% or less, 70% or less, 65% or less, or 60% or less.

The porous metal portion of the base component 30 may be a molded article of any metal. The porous metal portion may be a metal molded article containing, for example, nickel, chromium, tin, aluminum, iron, tungsten, iron, cobalt, copper, zirconium, titanium, silicon, magnesium, stainless steel, or an alloy, oxide or carbide containing these. A porous metal portion containing nickel or a nickel alloy can contribute to further improvement in efficiency of the gas-solid phase reaction.

The length L of the base component 30 (plate-like portion) in the direction along the axis X, the maximum width W in the direction perpendicular to the axis X, and the thickness T of the plate-like portion are appropriately set depending on the shape, size and the like of the reaction tube. The length L may be, for example, 5 mm or more or 40 mm or more, and may be 2,000 mm or less or 5,000 mm or less. The length L is often larger than the maximum width W, but it may be smaller than the maximum width W. The thickness of the base component 30 (plate-like portion) or the structure catalyst 3 may be, for example, 0.1 mm or more and 100 mm or less.

The maximum width W of the base component 30 (plate-like portion) is set so that the structure catalyst 3 can be inserted into the reaction tube 1. When the maximum width W is close to the inner diameter of the reaction tube, it is advantageous in terms of making the device compact, and the efficiency of heat transfer from the reaction tube can be increased. Specifically, the ratio of the maximum width W of the base component 30 to the inner diameter of the reaction tube 1 may be 0. 85 or more or 0. 90 or more, and may be 1.0 or less or 0. 95 or less. The maximum width W may be, for example, 2 mm or more or 5 mm or more, and may be 500 mm or less or 100 mm or less.

In the example of the structure catalyst shown in FIG. 2 and FIG. 3, the axis X, which is the center of twist, is positioned inside the base component 30. The base component 30 may have a structure in which the axis X is positioned outside the base component 30, and pores are formed along and in the vicinity of the axis X. However, in consideration of reducing the pressure loss of the gas passing through the structure catalyst and reaction efficiency, a structure in which the axis X is positioned inside the base component 30 can be advantageous.

**In** the case of the structure catalyst exemplified in FIG. 2, the plate-like portion of the base component is twisted in one direction around the axis X, but the shape of the base component (plate-like portion) is not limited thereto, and can be deformed appropriately. For example, the base component (plate-like portion) may have a portion that is twisted in a clockwise direction and a portion that is twisted in a counterclockwise direction when the base component is viewed in the direction along the axis X. The period of twist does not have to be constant, but may vary. **In** order to efficiently stir the gas, the base component (plate-like portion) may be a static mixer element. As will be understood by those skilled in the art, a base component containing a twisted plate-like article can be obtained by processing a molded article by a general method.

The catalyst layer 35 contains a catalyst that promotes the reaction of the gas-solid phase reaction. The type of the catalyst can be selected depending on the type of the gas-solid phase reaction and the like. For example, the catalyst may contain at least one catalyst metal selected from the group consisting of nickel, ruthenium, rhodium, potassium, calcium, sodium, and iridium. These catalyst metals function as catalysts that promote, for example, the methanation reaction.

The catalyst constituting the catalyst layer 35 may contain at least one catalyst metal selected from the group consisting of nickel, magnesium, zirconium, silicon, titanium, aluminum, ruthenium, and rhodium and at least one metal oxide selected from the group consisting of nickel oxide (NiO₂), aluminum oxide (Al₂O₃), zirconium oxide (ZrO₂), silicon oxide (SiO₂), magnesium oxide (MgO), and titanium oxide (TiO₂). Catalysts containing these components function as catalysts that promote, for example, the dry reforming reaction. The catalyst constituting the catalyst layer 35 may contain at least one catalyst metal selected from the group consisting of copper, nickel, zinc, aluminum, ruthenium, platinum, rhodium, silver, chromium, and magnesium, and at least one metal oxide selected from among zinc oxide (ZnO), aluminum oxide (Al₂O₃), chromium oxide (Cr₂O₃), silicon oxide (SiO₂), and magnesium oxide (MgO). Catalysts containing these components function as catalysts that promote, for example, the water-gas shift reaction and the reverse shift reaction.

The catalyst constituting the catalyst layer 35 may contain a carrier that supports a catalyst metal. The carrier contains, for example, a metal oxide containing at least one metal element selected from among cerium, zirconium, yttrium, aluminum, silicon, and magnesium. The metal oxide may contain two or more metal elements selected from among cerium, zirconium, yttrium, aluminum, silicon, and magnesium. The carrier may be cerium oxide (CeO₂), zirconium oxide (ZrO₂), yttrium oxide (Y₂O₃), aluminum oxide (Al₂O₃), silicon oxide (SiO₂), magnesium oxide (MgO), zinc oxide (ZnO), chromium oxide (Cr₂O₃, Cr₃O₄), iron oxide (Fe₂O₃, Fe₃O₄), or a composite oxide containing at least one metal element selected from among cerium, zirconium, yttrium, aluminum, silicon, magnesium, zinc, chromium, and iron. The composite oxide may contain two or more metal elements selected from among cerium, zirconium, yttrium, aluminum, silicon and magnesium.

The content of the catalyst metal in the catalyst layer 35 is not particularly limited, and may be, for example, 0. 01 to 50 mass% based on the mass of the carrier.

The total content of the carrier and catalyst metal in the catalyst layer 35 based on the mass of the catalyst layer 35 may be, for example, 0.1 to 100 mass%, 1 to 100 mass%, 10 to 100 mass%, 30 to 100 mass%, 50 to 100 mass%, 70 to 100 mass%, 80 to 100 mass%, or 90 to 100 mass%.

For example, the spiral-type structure catalyst can be produced by a method including preparing a catalyst dispersion containing a particulate catalyst and a dispersion medium, immersing a spiral-type base component having a porous metal portion in the catalyst dispersion, removing the base component from the catalyst dispersion, and removing the dispersion medium from the catalyst dispersion adhered to the base component. In the structure catalyst obtained by this method, a part of the catalyst layer often penetrates into the pores of the porous metal portion. The dispersion medium contained in the catalyst dispersion may be water, an alcohol or a combination thereof.

### [Examples]

The present invention is not limited to the following examples.

### Test 1A: Methanation reaction

### 1-1. Structure catalyst

### (1) Spiral-type base component

The following porous metal substrate, which is a plate-like molded article made of a nickel-chromium alloy, and a non-porous aluminum substrate were prepared as base components.

### Porous metal substrate:

effective area (specific surface area): 1,200 to 6,000 m²/m³, width: 11 mm, length: 150 mm, thickness: 1.2 mm, average pore diameter: 0.5 to 1.0 mm, porosity: 60 to 98%

### Aluminum substrate:

width: 11 mm, length: 150 mm, thickness: 1.0 mm

Both ends of each base component were gripped, and the base component was twisted in a direction of rotation about an axis along the longitudinal direction of the base component. Accordingly, a porous Ni-Cr member and an aluminum member were obtained as a spiral-type base component having a porous metal portion.

### (2) Formation of catalyst layer

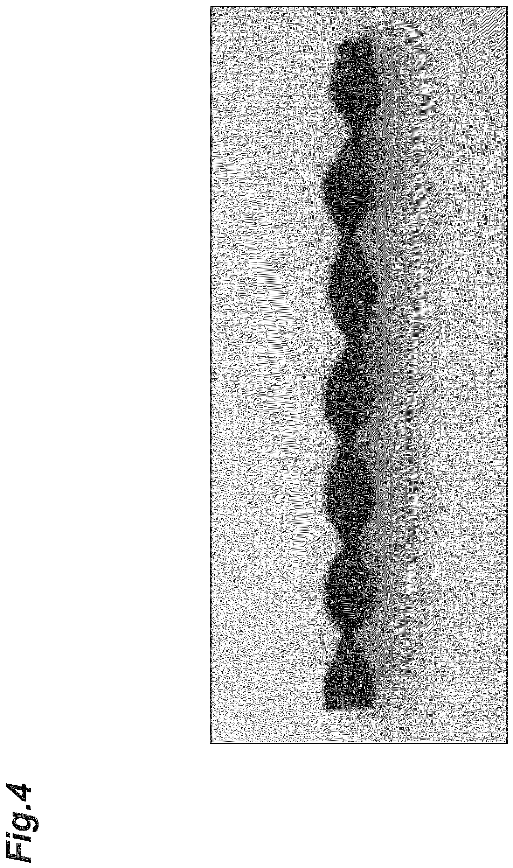

Each base component was immersed in a sodium hydroxide aqueous solution. Each base component removed from the sodium hydroxide aqueous solution was immersed in a catalyst slurry (catalyst dispersion) in which Ni-supported CeO₂ particles (Ni/CeO₂) were dispersed in water at room temperature for about 0.5 to 10 minutes. The catalyst slurry adhered to the base component removed from the catalyst slurry was dried with hot air to obtain a structure catalyst having a catalyst layer containing Ni/CeO₂ and covering the outer surface of the base component. The amount of the catalyst layer adhered per base component was 1.1 g for the porous Ni-Cr member and 0.33 g for the aluminum member. The amount of the catalyst layer adhered per 1 L of the volume of the base component was 78 g/L for the porous Ni-Cr member and 23 g/L for the aluminum member. FIG. 4 is an image of an example of the produced structure catalyst.

A catalyst slurry in which Ru-supported CeO₂ particles (Ru/CeO₂) were dispersed in water was prepared, and in the same procedure as above, a structure catalyst having a catalyst layer containing Ru/CeO₂ and covering the outer surface of the base component was obtained. The amount of the catalyst layer adhered per metal member was 1.1 g for the porous Ni-Cr member and 0.33 g for the aluminum member. The amount of the catalyst layer adhered per 1 L of the volume of the base component was 78 g/L for the porous Ni-Cr member and 23 g /L for the aluminum member.

### 1-2. Methanation reaction test

### Example 1A

Three structure catalysts each having a porous Ni-Cr member and a catalyst layer containing Ni/CeO₂ were inserted in series into a first reaction tube (inner diameter: 12 mm) having a gas inlet and a gas outlet. Three structure catalysts each having a porous Ni-Cr member and a catalyst layer containing Ru/CeO₂ were inserted in series into a second reaction tube (inner diameter: 12 mm) connected to the gas outlet of the first reaction tube via a pipe. A raw material gas containing carbon dioxide and hydrogen, and oxygen gas were continuously introduced through the gas inlet of the first reaction tube. The gas discharged from the first reaction tube was introduced into the second reaction tube together with additional oxygen gas. The CO₂ concentration in the product gas discharged from the second reaction tube was quantified, and the conversion of CO₂ to methane (CO₂ conversion) was determined based on the measurement results. The methanation reaction test was performed under conditions in which the first reaction tube and the second reaction tube were heated in an electric furnace at 200°C or 100°C and under conditions in which the reaction tubes were not heated in an electric furnace while changing the concentration of carbon dioxide in the raw material gas. Other reaction conditions were as follows.
Gas flow rate: 3.0 L/min
H₂/CO₂ ratio: 4.0 (stoichiometry)
CO₂ concentration: 1 to 16 vol%
O₂ concentration: 3 vol% (the first reaction tube and the second reaction tube)

### Comparative Example 1A

A reaction test was performed in the same manner as in Example 1 except that three structure catalysts each having an aluminum member and a catalyst layer containing Ni/CeO₂ were inserted into the first reaction tube, and three structure catalysts each having an aluminum member and a catalyst layer containing Ru/CeO₂ were inserted into the second reaction tube.

### Results

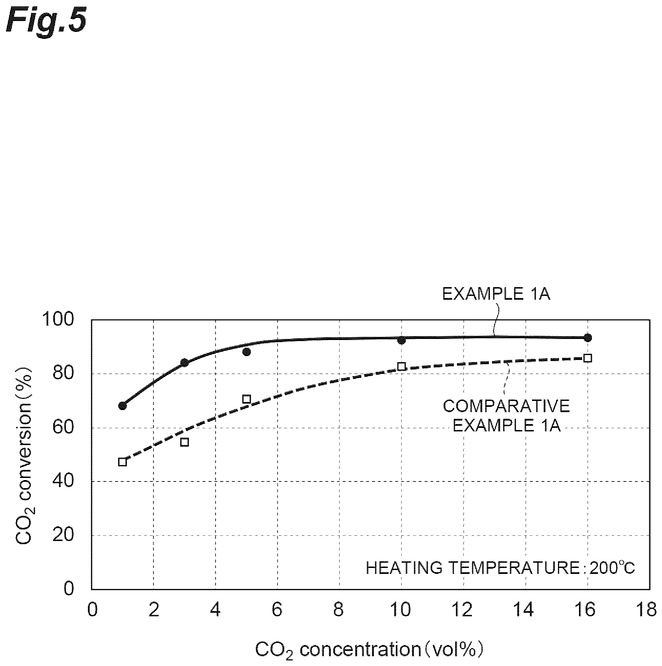

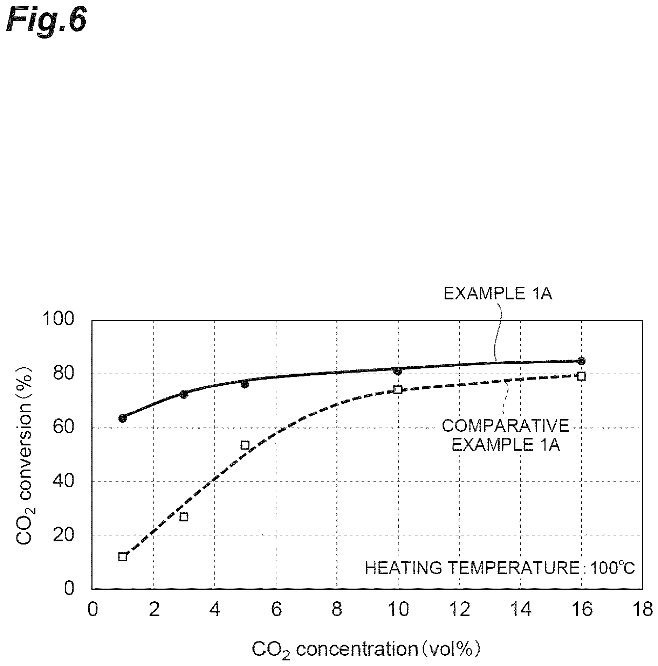

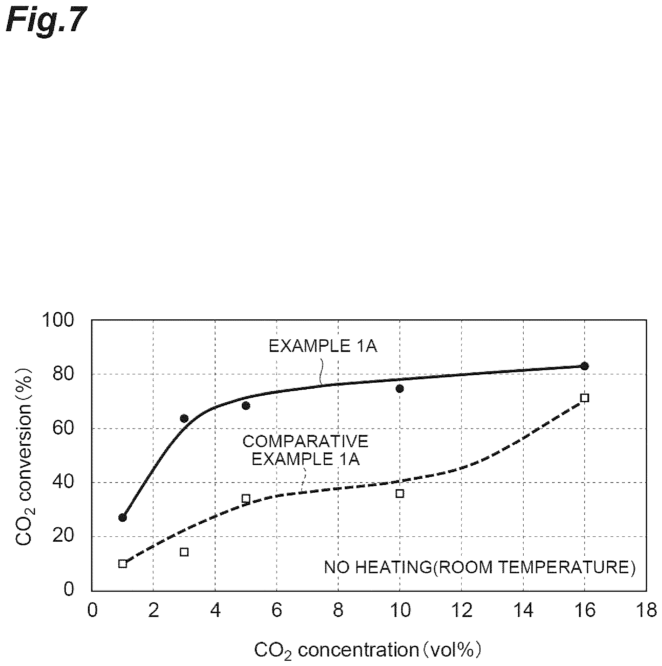

FIG. 5, FIG. 6 and FIG. 7 are graphs showing the relationship between the CO₂ conversion and the CO₂ concentration in the raw material gas. FIG. 5 shows the results under reaction conditions in which the heating temperature was 200°C, FIG. 6 shows the results under reaction conditions in which the heating temperature was 100°C, and FIG. 7 shows the results under reaction conditions in which the reaction tubes were not heated. The structure catalyst having a spiral-type porous Ni-Cr member (Example 1A) exhibited a significantly higher CO₂ conversion than the structure catalyst having a spiral-type non-porous aluminum member (Comparative Example 1A). The difference between the two structure catalysts was particularly large under reaction conditions without heating in an electric furnace. The effect confirmed here exceeded the level that could be expected solely from an increase in the catalyst amount, and can be said to be exhibited through a specific action resulting from the combination of the porous metal member and the spiral-type shape.

### Test 1B: Methanation reaction

### 1-1. Structure catalyst

### (1) Spiral-type base component

The following porous metal substrate, which is a plate-like molded article made of a nickel-chromium alloy, and a non-porous aluminum substrate were prepared as base components.

### Porous metal substrate:

effective area (specific surface area): 1,000 to 7,000 m²/m³, width: 7 mm, length: 50 mm, average pore diameter: 0. 4 to 1.0 mm

### Aluminum substrate:

width: 7 mm, length: 50 mm

Both ends of each base component were gripped, and the base component was twisted in a direction of rotation about an axis along the longitudinal direction of the base component. Accordingly, a porous Ni-Cr member and a non-porous aluminum member were obtained as a spiral-type base component having a porous metal portion.

### (2) Formation of catalyst layer

Using a porous Ni-Cr member or a non-porous aluminum member as a base component, and a catalyst layer containing Ru/CeO₂ and covering the outer surface of the base component was formed in the same method as in the test 1A to obtain a structure catalyst. The content of Ru in Ru/CeO₂ based on the mass of Ru/CeO₂ was 10 mass%. In both the porous Ni-Cr member and the non-porous aluminum member, the amount of the catalyst layer adhered per base component was 0.15 g.

### 1-3. Granular catalyst

A granular catalyst was prepared by diluting the following catalyst composed of Ru-supported CeO₂ particles with quartz sand.
Catalyst: Ru/CeO₂ (Ru content: 10 mass% based on the mass of Ru/CeO₂)
Average particle diameter: 0.5 mm
Catalyst amount: 0.6 g

### 1-2. Methanation reaction test

### Example 1B

Four structure catalysts each having a porous Ni-Cr member and a catalyst layer containing Ni/CeO₂ were inserted in series into a reaction tube (inner diameter: 8 mm) having a gas inlet and a gas outlet. The total length of the four structure catalysts was 200 mm. A raw material gas containing carbon dioxide, hydrogen, nitrogen and oxygen was continuously introduced through the gas inlet of the reaction. The CO₂ concentration in the product gas discharged from the reaction tube was quantified and the conversion of CO₂ to methane (CO₂ conversion) was determined based on the measurement results. The methanation reaction test was performed under conditions in which the reaction tube was heated in an electric furnace set to 150°C, 200°C, 250°C, 300°C, 350°C or 400°C. The flow rates of the gases were as follows. The pressure on the upstream side from the structure catalyst and the pressure on the downstream side from the structure catalyst were measured, and the difference between the pressures was defined as a pressure loss ΔP.
Total gas flow rate: 1,000 mL/min
CO₂ flow rate: 100 mL/min (10 vol%)
H₂ flow rate: 460 mL/min (46 vol%)
N₂ flow rate: 410 mL/min (41 vol%)
O₂ flow rate: 30 mL/min (3 vol%)

### Comparative Example 1B

A reaction test was performed in the same manner as in Example 1B except that four structure catalysts each having an aluminum member and a catalyst layer containing Ni/CeO₂ were inserted into the reaction tube.

### Comparative Example 2B

In a reaction tube with the same size as in Example 1B, a granular catalyst containing Ni/CeO₂ was placed and a catalyst layer with a length of 200 mm was formed. The amount of Ni/CeO₂ contained in the catalyst layer was 0.6 g. A methanation reaction test using the catalyst layer was performed under the same conditions as in Example 1B. The pressure on the upstream side from the catalyst layer and the pressure on the downstream side from the catalyst layer were measured, and the difference between the pressures was defined as a pressure loss ΔP.

### Results

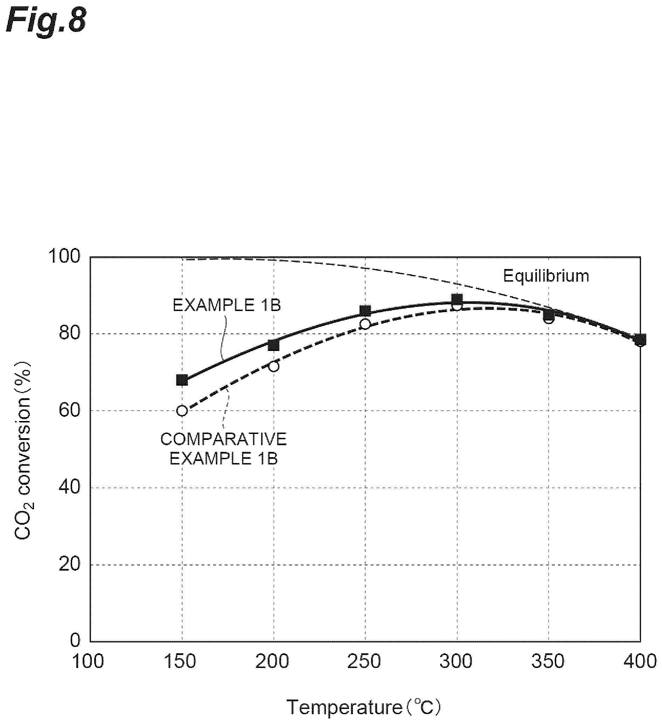

FIG. 8 is a graph showing the relationship between the CO₂ conversion and the set temperature of the electric furnace. "Equilibrium" in the drawing indicates the CO₂ conversion in the equilibrium state at each temperature. The structure catalyst having a spiral-type porous Ni-Cr member (Example 1B) exhibited a significantly higher CO₂ conversion than the structure catalyst having a spiral-type non-porous aluminum member (Comparative Example 1B). The difference between the two structure catalysts was particularly large under reaction conditions at a low temperature of 150 to 250°C.

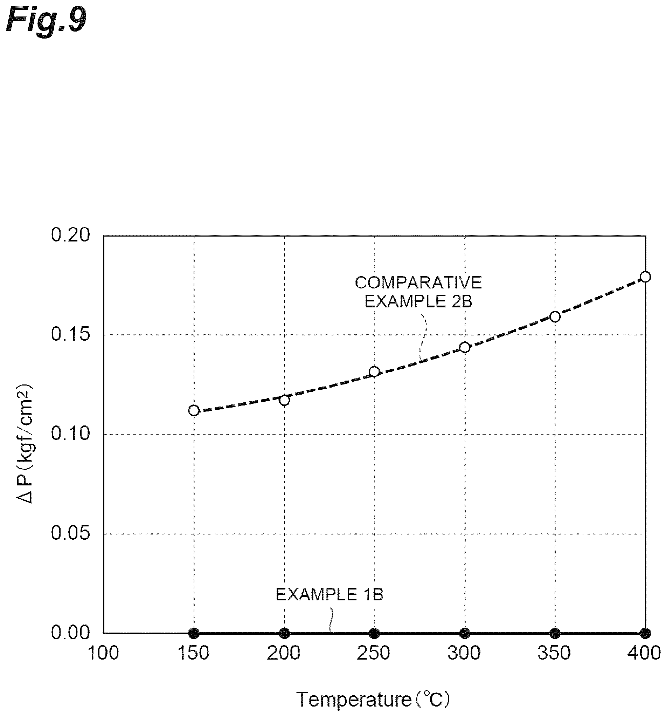

FIG. 9 is a graph showing the relationship between the pressure loss ΔP and the set temperature of the electric furnace. In the case of the catalyst layer of Comparative Example 2B containing a granular catalyst, the pressure loss tended to increase as the temperature increased. On the other hand, in the case of Example 1B using the spiral-type structure catalyst, practically no pressure loss was observed regardless of the temperature.

### Test 2: Dry reforming reaction

### 2-1. Structure catalyst

### (1) Spiral-type base component

The following porous metal substrate, which is a plate-like molded article made of a nickel-chromium alloy, and a non-porous stainless steel substrate were prepared as base components.

### Porous metal substrate:

effective area (specific surface area): 1,200 to 1,500 m²/m³, width: 7 mm, length: 55 mm, thickness: 2.2 mm, average pore diameter: 0.85 mm

### Stainless steel substrate:

width: 7 mm, length: 55 mm, thickness: 0.1 mm

Both ends of each base component were gripped, and the base component was twisted in a direction of rotation about an axis along the longitudinal direction of the base component. Accordingly, a porous Ni-Cr member and a stainless steel member were obtained as a spiral-type base component having a porous metal portion.

### (2) Formation of catalyst layer

Each base component was immersed in a sodium hydroxide aqueous solution. Each base component removed from the sodium hydroxide aqueous solution was immersed in a catalyst slurry (catalyst dispersion) in which Ni-supported Al₂O₃ particles (Ni/Al₂O₃) were dispersed in water at room temperature for about 0.56 to 5 minutes. The catalyst slurry adhered to the base component removed from the catalyst slurry was dried with hot air to obtain a structure catalyst having a catalyst layer containing Ni/Al₂O₃ and covering the outer surface of the base component. The amount of the catalyst layer adhered per base component was 93.9 mg for the porous Ni-Cr member and 20.6 mg for the stainless steel member. The amount of the catalyst layer adhered per 1 L of the volume of the base component was 45 g/L for the porous Ni-Cr member and 10 g/L for the stainless steel member.

### 2-2. Dry reforming reaction test

### Example 2

Six structure catalysts each having a porous Ni-Cr member and a catalyst layer containing Ni/Al₂O₃ were inserted in series into a reaction tube (inner diameter: 8 mm) having a gas inlet and a gas outlet. A raw material gas containing methane and carbon dioxide was continuously introduced through the gas inlet of the reaction tube. The concentrations of methane and carbon dioxide in the product gas discharged from the reaction tube were quantified, and the conversions of methane and carbon dioxide were determined based on the measurement results. The dry reforming reaction test was performed under conditions in which the reaction tube was heated in an electric furnace at 550, 600°C, 650°C, or 700°C. Other reaction conditions were as follows.
Gas flow rate: 1,000 mL/min
CO₂/CH₄ ratio: 1.2

### Comparative Example 2

A reaction test was performed in the same manner as in Example 2 except that six structure catalysts each having a stainless steel member and a catalyst layer containing Ni/Al₂O₃ were inserted into the reaction tube.

### Results

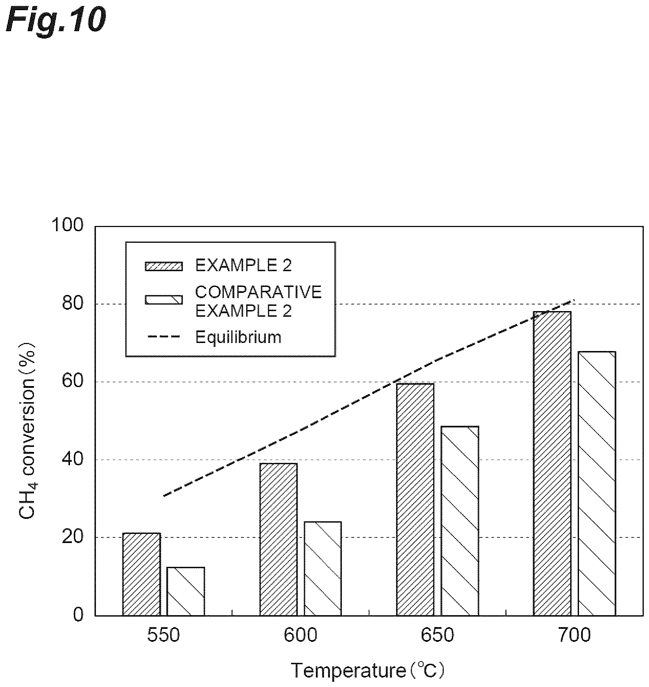

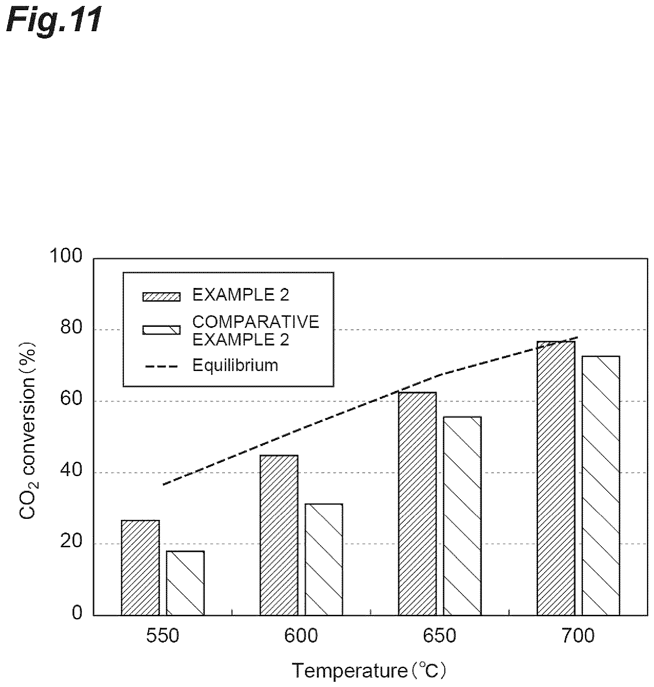

FIG. 10 is a graph showing the relationship between the methane conversion (CH₄ conversion) and the temperature (the set temperature of the electric furnace). FIG. 11 is a graph showing the relationship between the carbon dioxide conversion (CO₂ conversion) and the temperature (the set temperature of the electric furnace). "Equilibrium" in each drawing indicates the CH₄ or CO₂ conversion in the equilibrium state at each temperature. The structure catalyst having a spiral-type porous Ni-Cr member (Example 2) exhibited a significantly higher CH₄ conversion and CO₂ conversion than the structure catalyst having a spiral-type non-porous stainless steel member (Comparative Example 2).

### Test 3: Reverse shift reaction

### 3-1. Structure catalyst

### (1) Spiral-type base component

The following porous metal substrate, which is a plate-like molded article made of a nickel-chromium alloy, and a non-porous stainless steel substrate were prepared as base components.

### Porous metal substrate:

effective area (specific surface area): 3,000 to 6,000 m²/m³, width: 7 mm, length: 100 mm, thickness: 1.2 mm, average pore diameter: 0.51 mm, porosity: 60 to 98%

### Aluminum substrate:

width: 7 mm, length: 100 mm, thickness: 1.0 mm

Both ends of each base component were gripped, and the base component was twisted in a direction of rotation about an axis along the longitudinal direction of the base component. Accordingly, a porous Ni-Cr member and an aluminum member were obtained as a spiral-type base component having a porous metal portion.

### (2) Formation of catalyst layer

Each base component was immersed in a sodium hydroxide aqueous solution. Each base component removed from the sodium hydroxide aqueous solution was immersed in a catalyst slurry (catalyst dispersion) in which Ru-supported CeO₂ particles (Ru/CeO₂, Ru: 10 mass%) were dispersed in water at room temperature for about 0.5 to 5.0 minutes. The catalyst slurry adhered to the base component removed from the catalyst slurry was dried with hot air to obtain a structure catalyst having a catalyst layer containing Ru/CeO₂ and covering the outer surface of the base component. The amount of the catalyst layer adhered per base component was 1.2 g for the porous Ni-Cr member and 0.4 g for the aluminum member. The amount of the catalyst layer adhered per 1 L of the volume of the base component was 160 g/L for the porous Ni-Cr member and 80 g/L for the aluminum member.

### 3-2. Reverse shift reaction test

### Example 3

Two structure catalysts each having a porous Ni-Cr member and a catalyst layer containing Ru/CeO₂ were inserted in series into a reaction tube (inner diameter: 8 mm) having a gas inlet and a gas outlet. A raw material gas containing carbon dioxide, hydrogen and nitrogen was continuously introduced through the gas inlet of the reaction tube. The concentration of carbon dioxide in the product gas discharged from the reaction tube was quantified, and the conversion of carbon dioxide was determined based on the measurement results. The dry reforming reaction test was performed under conditions in which the reaction tube was heated in an electric furnace at 400, 450°C, 500°C, 550°C, or 600°C. Other reaction conditions were as follows.
Gas flow rate: 500 mL/min
CO₂/H₂/N₂ ratio: 1/1/0.5

### Comparative Example 3

A reaction test was performed in the same manner as in Example 3 except that two structure catalysts each having an aluminum member and a catalyst layer containing Ru/CeO₂ were inserted into a reaction tube.

### Results

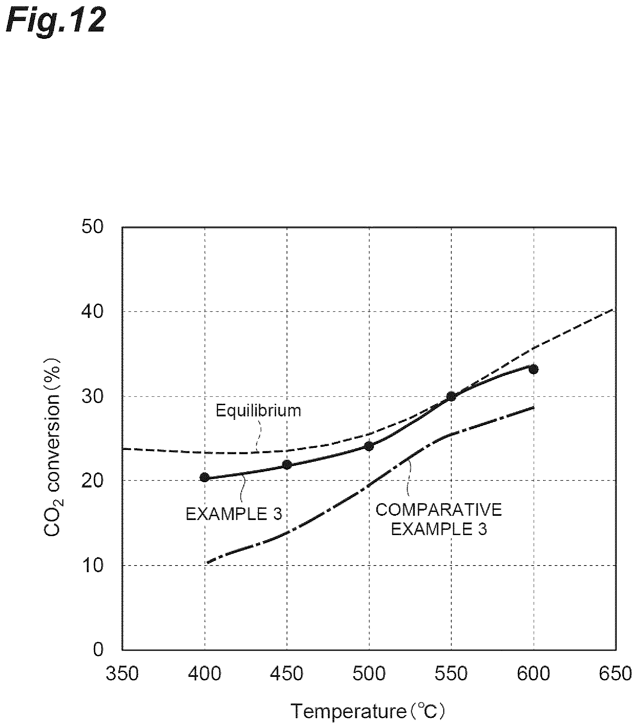

FIG. 12 is a graph showing the relationship between the carbon dioxide conversion (CO₂ conversion) and the temperature (the set temperature of the electric furnace). "Equilibrium" in the drawing indicates the CO₂ conversion in the equilibrium state at each temperature. The structure catalyst having a spiral-type porous Ni-Cr member (Example 3) exhibited a significantly higher CO₂ conversion than the structure catalyst having a spiral-type non-porous aluminum member (Comparative Example 3).

### Reference Signs List

1 Reaction tube, 3 Structure catalyst, 7 Heat source, 10 Reaction device, 30 Base component (plate-like portion), 35 Catalyst layer, G₀ Raw material gas, S Outer surface, X Axis

## Claims

1. A structure catalyst, comprising:
a base component that extends along a fixed axis; and
a catalyst layer that is provided on the base component,
wherein the base component has a porous metal portion including an outer surface of the base component, and the catalyst layer is adhered to the porous metal portion, and
the base component has a plate-like portion that extends along the axis while being twisted in a direction of rotation about the axis.

2. The structure catalyst according to claim 1, wherein a mass of the catalyst layer per 1 L of a volume of the base component is 1 g or more.

3. The structure catalyst according to claim 1, wherein an average pore diameter of the porous metal portion is 0.1 mm or more.

4. The structure catalyst according to claim 1, wherein the catalyst layer covers an entire outer surface of the base component.

5. A method for producing a compound, comprising producing a desired compound from a raw material gas through a gas-solid phase reaction in a reaction tube in which the structure catalyst according to any one of claims 1 to 4 is provided,
wherein the structure catalyst is inserted into the reaction tube in an orientation such that the axis is parallel to a longitudinal direction of the reaction tube.

6. A reaction device, comprising:
a reaction tube; and
the structure catalyst according to any one of claims 1 to 4 accommodated in the reaction tube,
wherein the structure catalyst is inserted into the reaction tube in an orientation such that the axis is parallel to a longitudinal direction of the reaction tube.
